# EUROPEAN PATENT SPECIFICATION

(11) **EP 4 611 536 B1**
(45) Date of publication and mention of the grant of the patent: **01.07.2026**
(21) Application number: 23798262.4
(22) Date of filing: 01.11.2023
(51) Int. Cl.: A01K 45/00, A01K 43/00

(54) **METHOD FOR DETERMINING THE GENDER OF A CHICK**
VERFAHREN ZUR BESTIMMUNG DES GESCHLECHTS EINES KÜKENS
PROCÉDÉ DE DÉTERMINATION DU SEXE D'UN POUSSIN

(30) Priority: 03.11.2022 NL 2033456
(43) Date of publication of application: 10.09.2025
(73) Proprietor: HatchTech Group B.V., 6745 XW De Klomp (NL)
(72) Inventor: METER, Tjitze, 3911 ET Rhenen (NL)
(74) Representative: Nederlandsch Octrooibureau
(86) International application number: PCT/EP2023/080469
(87) International publication number: WO 2024/094751

(56) References cited:
- US-A1- 2005 206 876
- US-A1- 2022 196 624

## Description

### Field of the invention

The present invention relates to a method for determining the gender of a birds in ovo of a plurality of eggs. In particular, the plurality of eggs concerns a high volume throughput situation like millions of eggs in a supply chain including laying hens and ultimately consumption eggs.

### Background art

Commercial birds for laying eggs are generally sorted by gender after hatch. The sorting by gender has long been and often still is typically carried out by hand by visual inspection. This manual process is a time-consuming, tedious, and inaccurate process. Since the failure to properly sort birds by gender can lead to problems in poultry or egg production, it would be extremely useful to have a reliable means for sorting birds by gender without the need for visual inspection of the bird. Therefore from US 2003/0096319 A1 a method is known of determining the gender of a bird in ovo. The method of US 2003/0096319 A1 detects the presence or absence of an elevated level of a sex-related hormone in the extra-embryonic fluid of a bird egg; and then the gender of the bird within the egg is determined from the presence of an elevated level of said sex-related hormone in said extra-embryonic fluid. However, known methods of determining the gender of a bird in ovo, are not accurate enough to make a viable business case.

US 2022/196624 A1 discloses a system for determining in non-invasive manner the gender of a fertile egg, in particular poultry eggs.

US 2005/206876 A1 discloses a method and apparatus for determining the viability of eggs laid by egg-laying animals.

### Summary of the invention

The present invention seeks to provide method for determining the gender of a birds in ovo of a plurality of eggs, which method provides more accurate results.

The present invention seeks to provide a method for determining the gender of a birds in ovo of a plurality of eggs, wherein a problem with a known method is at least partly solved.

The present invention seeks to provide an alternative method for determining the gender of a birds in ovo of a plurality of eggs.

The invention is set out in the appended claims. According to the present invention, a method for determining the gender of a birds in ovo of a plurality of eggs is provided, in which the method comprises a first test, and a second test separate and different from the first test, each of the first and second test comprising the steps of examining a bird egg and determining the gender of the bird within the egg, and wherein the method further comprises the step of determining a test usability value for each individual egg of the plurality of eggs and deciding a test sequence for each individual egg based on the test usability value, the test sequence comprising one or both of the first and second test, and separating said plurality of eggs into at least a first and second subset of eggs based on gender.

Because of the method comprising a first and second test, the method provides the ability to optimize the test or test sequence for an individual egg while the step of determining a test usability value helps to decide on a test sequence for each individual egg. As a consequence, the accuracy of the result is improved, like for example better than 98%, or even better than 99,5% accurate while known methods are accurate for less than 98%, like between 95% to 98% of the cases. The accuracy of the result is very important to be able to make a viable business case. Also, typically determining the gender of birds in ovo concerns a high volume throughput of valuable fertilized eggs which makes accuracy of the result all the more important.

Based on the test usability value, a first decision can be made to start with the first test or the second test. When starting with the first test, the first test result can provide a basis as well for the test usability value, or better an updated test usability value for an individual egg based on the first result. Following the updated test usability value, a decision can be made to perform the second test or perform no further test at all. When the decision is made to perform no further test at all, an undetermined status or reject status will be assigned to the respective egg.

The second test being separate and different from the first test means that the first and second test are separated in time and normally also space from perspective of an individual egg under test. The first and second test also differ in working principle.

The claim requirement that "each of the first and second test comprise the steps of examining a bird egg and determining the gender of the bird within the egg" does not exclude that the first and second test may have a method step or steps in common. However, it must be clear that the first and second test each end up with a respective first and second result wherein the first and second result are independent.

In practice, in a practical implementation of the current invention in industry, all viable eggs will be tested for gender. However the requirement of determining a test usability value for each individual egg of the plurality of eggs does not exclude that some eggs may not be tested for gender or have a test usability value determined. However, in practice, there is always a plurality of egg that are all tested.

The step of separating said plurality of eggs into at least a first and second subset of eggs based on gender can be done directly after the step of determining the gender of the bird within the egg. However, it will be clear that separating into subsets of eggs based on gender can be done separated in space and time from the actual determination of the gender of the bird within the egg.

According to the invention, the first and second test at least differ in working principle. This all the more improves the accuracy of the result of the method.

In an embodiment of the method according to the invention, the step of determining a test usability value for an individual egg precedes the first test and the second test, and a decision is made to perform the first test or the second test or reject the individual egg based on the usability value, or the step of determining a test usability value for an individual egg precedes the second test, and a decision is made to perform the second test or reject the individual egg based on the usability value. This saves test capacity since unnecessary execution of the first test or second test is avoided. The test usability value can also be referred to as to "fitness for use" of an egg for a particular test providing a test result with an acceptable degree of accuracy.

In an embodiment of the method according to the invention, the first test provides a first result and the second test provides a second result and wherein the determining the test usability value for an individual egg is at least based on the first result, and a decision is made to perform the second test or not based on the usability value. The use of a first test result to determine the test usability value improves the quality of the usability value. This in turn can provide a potential for better use of test capacity.

According to the invention, the determining the test usability value for each individual egg of the plurality of eggs comprises assessing an individual egg for providing an egg assessment and wherein the test usability value for the individual egg is at least based on the egg assessment. The use of an egg assessment to determine the test usability value improves the quality of the usability value even more. It will be clear that the step of assessing a an individual egg for providing an egg assessment differs with the first and second test at least therein that no determination of the gender of the bird within the egg results from the egg assessment. The only purpose of the egg assessment is to contribute to the quality of the test usability value for the individual egg. As a result, a better decision can be made on a test sequence for an individual egg.

In an embodiment, the method comprises updating the test usability value to an updated test usability value based on the first result and/or the second test result. This provides even more a potential for better use of test capacity since not useful executions of at least the second test can be avoided.

According to the invention, the step of assessing an individual egg comprises one or more of; Colori metering, weighing, temperature measuring, imaging, candling, measuring an egg shell thickness. This way, the usability of the first and second test can be determined in a more accurate way while the integrity of the egg is maintained and intrusion of the egg is not required in the context of the egg assessment. It will be clear that a step of imaging may involve use of electromagnetic waves in and/or outside the visible spectrum of the human eye.

In an embodiment, the method according to the invention comprises a step of sorting eggs based on test sequence and performing different test sequences in parallel. The sorting of eggs based on test sequence facilitates to perform different test sequences in parallel. In turn, performing different test sequences in parallel, improves the throughput of the plurality of eggs to be tested. In addition, this facilitates to maintain single stage batches of eggs, that is eggs in the same phase of development of an egg.

In an embodiment of the method according to the invention the first and second test differ in one or more of; cost, consumables, cycle time, and lead time. In particular, the first test is a fast and low cost test while the second test may be slower and more expensive. This still results in a viable business case when prognosing that only the first test suffices for the majority of eggs like more than 90% of the plurality of eggs.

In an embodiment of the method the first test and/or the second comprises one or more of spectroscopy, component analyses, and image analysis.

In an embodiment of the method the first test comprises spectroscopy and the second test comprises a PCR test. At least the first test may also involve imaging technique and it will be clear that any suitable test method, that is working principle, for the first and second test is conceivable.

In an embodiment of the method according to the invention the first and/or second test comprises detecting the presence or absence of an elevated level of a sex-related component in the bird egg, and determining the gender of the bird within the egg from the presence of an elevated level of said sex-related component in the egg. In particular the presence or absence of an elevated level of a sex-related component is determined in the extra-embryonic fluid of the bird egg. It will be clear that any suitable egg compartment or component is conceivable. The component can be a hormone or any other suitable matter that has a clear and consistent relation with the gender of a bird in an egg.

In an embodiment of the method according to the invention the first and/or second test comprises examining DNA for determining the gender of the bird within the egg. The DNA can be examined in any suitable manner like based on an optical examination method and/or with a Polymerase chain reaction (PCR).

In an embodiment, the method according to the invention comprises the step of sampling an egg to provide an amount of extra-embryonic fluid, and using the amount for the first and second test. The sample can be used only for the first test in case the second test is not required. And although the first and second test differ, still it is conceivable that the one and the same sample is used, or partly used, for both the first test and the second test. This way, the integrity of the egg is maintained as much as possible. The amount of extra-embryonic fluid can be taken from the allantoic fluid, however any suitable fluid will suffice.

In an embodiment, the method according to the invention comprises synchronizing total lead times for test sequences, preferably all test sequences, for maintaining batches of eggs before and after the method. Maintaining batches of eggs is beneficial to assure a predictable hatching window for a batch of eggs, since eggs at a similar development phase can still be hatched together. The hatching window is a period of time, usually about 48 hours, wherein all the eggs of a batch hatch. In this context there are three possible test sequences being the first test, the first and second test, and only the second test. It is conceivable to introduce buffers to temporarily store eggs in order to synchronize total lead times for test sequences.

In an embodiment, the method according to the invention comprises hatching a batch of eggs, wherein the batch contains respective eggs that were subjected to different respective test sequences.

In an embodiment, the method according to the invention comprises performing the method with embryonated eggs after day 4 in their development, in particular at about day 9 in their development. This improves the accuracy of the determination of the gender of a bird in ovo and still avoids at the same time pain perception by the bird in ovo in case of processing a rejected egg.

In an embodiment of the method according to the invention, the first test and the second test are executed on different moments in the development of a bird egg, in particular for a chicken egg the first test is executed before day 11 and the second test is executed after day 11 in the development of the chicken egg. Executing the first test and the second test on different moments in the development of a bird egg enables to optimize the accuracy of both the first test and the second test.

In an embodiment of the method according to the invention, the second test is executed after day 16 in the development of the chicken egg, in particular after day 18 in the development of the chicken egg. This enables to optimize the accuracy of at least the second test all the more. Moreover, this enables to combine the second test with a step of handling the chicken eggs, like the transfer of eggs to a hatcher where the chicken eggs will hatch.

In an embodiment of the method according to the invention, the first test and the second test are executed on different moments in the development of a bird egg, and a time lapse between the first test and the second test exceeds 24 hours. This enables to optimize the accuracy of both the first test and the second test.

In an embodiment, the method according to the invention comprises climatizing a test environment, comprising maintaining the test environment at a temperature of about 35°. This avoids any adverse effect on the development of an bird egg as much as possible.

### Short description of drawings

The present invention will be discussed in more detail below, with reference to the attached drawing, in which
Fig. 1 shows a schematic overview of a first embodiment of the method according to the invention,
fig. 2 shows a schematic overview of a second embodiment of the method according to the invention, and
fig. 3 shows a schematic overview of a third embodiment of the method according to the invention.

### Description of embodiments

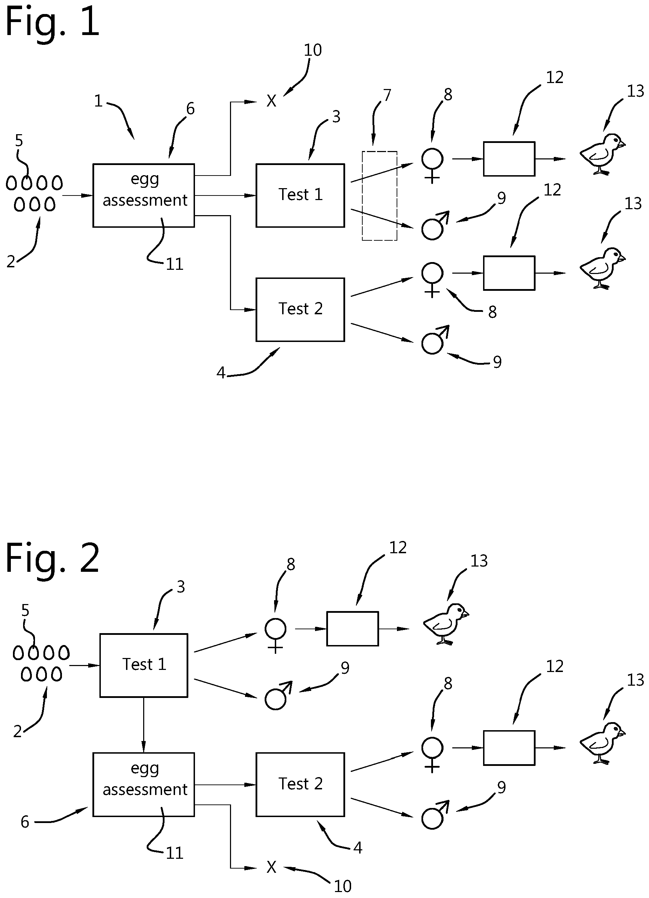

Fig. 1 shows a schematic overview of the method according to the invention. A method 1 is shown for determining the gender of a birds in ovo of a plurality of eggs 2. The method 1 comprises a first test 3 as well as a second test 4. The second test 4 is separate and different from the first test 3. Importantly, each of the first 3 and second test 4 comprises the steps of examining a bird egg 5 and determining the gender of the bird 13 within the egg 5. Thus the first test 3 as well as the second test 4 independently provide a respective first and second result that both indicate a gender of the bird 13 in the egg under test.

The method can be performed with embryonated eggs after day 4 in their development, in particular at about day 9 in their development. In case that the result of the first test 3 is not satisfactory and the second test 4 is required, it is conceivable that the first test 3 and the second test 4 are executed on different moments in the development of a bird egg 5. Like for example a time lapse between the first test 3 and the second test 4 may exceed 24 hours. In another example, for a chicken egg 5 the first test is executed on or before day 11 and the second test is executed after day 11 in the development of the chicken egg 5.

The method 1 is executed during development of the plurality of embryonated eggs 2. Therefore, the method may comprise climatizing a test environment, comprising maintaining the test environment at a temperature of about 35°. This is beneficial for the development of the plurality of the eggs 2.

The first test 3 and/or the second test 4 can be based on one or more of spectroscopy, component analyses, and image analysis. The image analyses may concern the appearance of an egg 5 under test. However, also inner structures of an egg 5 under test may be subjected to image analyses and contribute to a test result. The first 3 and second test 4 differ in one or more of; cost, consumables, working principle, cycle time, and lead time. In this case, the first test 3 is a low cost and fast test based on spectroscopy and the second test 4 is based on a relative slow PCR process that is also more expensive because of consumables that are involved.

The method 1 may further comprise the step of sampling an egg 5 in the context of the first test 3 and/or second test 4. The step of sampling an egg 5 provides an amount of extra-embryonic fluid, in particular allantoic fluid. The amount of fluid associated with an egg can be used for one of the first 3 and second test 4. It is however conceivable that the amount of extra-embryonic fluid is used for both the first and second test 4 in case the test sequence comprises both of the first 3 and second test 4.

The first 3 and/or second test 4 may comprise detecting the presence or absence of an elevated level of a sex-related component in the bird egg 5. In particular the amount of extra-embryonic fluid of the bird egg 5 can be used for detecting the presence or absence of an elevated level of a sex-related component in the bird egg 5. As a result, the gender of the bird 13 within the egg 5 can be determined from the presence of an elevated level of said sex-related hormone in the amount of extra-embryonic fluid.

The first 3 and/or second test 4 may comprise examining DNA for determining the gender of the bird 13 within the egg 5. In particular the amount of extra-embryonic fluid of the bird egg 5 can be used for examining DNA for determining the gender of the bird 13 within the egg 5. It will be clear that any suitable egg component is conceivable for the purpose of determining the gender of the bird 13 within the egg 5.

The method 1 further comprises the step 6 of determining a test usability value for each individual egg 5 of the plurality of eggs 2. The step 6 of determining the test usability value for each individual egg of the plurality of eggs 2 comprising assessing an individual egg for providing an egg assessment 11. In other words, the test usability value for the individual egg is at least based on the egg assessment 11 of that individual egg.

Here, the test usability value applies to the first test 3 as well as the second test 4. This means that based on the test usability value, the fitness of the egg under test for the first test 3 as well as the second test 4 is known.

The method 1 further comprises the step of separating 7 said plurality of eggs 2 into at least a first 8 and second 9 subset of eggs based on gender. Here the first subset 8 contains female chicks and the second subset 9 of eggs contains male chick. In this case, a third subset 10 is formed of eggs 5 of which the gender of the respective chicken embryo could not be determined.

In this case, the step 6 of determining a test usability value for an individual egg precedes the first test 3. Based on a test usability value, a decision is made then to perform the first test 3 or the second test 4. Here, the step of determining a test usability value for an individual egg precedes both the first test 3 and the second test 4, and a decision is made to perform the first test or the second test or reject the individual egg based on the usability value. This enables to proceed directly with the second test 4 without the need to do the first test 3.

The method 1 may include the step 11 of assessing an individual egg 5 for providing an egg assessment. Subsequently, the step of determining 6 the test usability value for the individual egg 5 is at least based on the egg assessment.

Here it is shown that the step 11 of assessing an individual egg 5 precedes the first test 3. The step 11 of assessing an individual egg 5 comprises one or more of; Colori metering, weighing, temperature measuring, imaging, candling, measuring an egg shell thickness.

The method 1 comprises the step of deciding on a test sequence for an individual egg 5 comprising one or more of the first test 3 and the second test 4. The decided test sequence depends on the test usability value. Based on the test usability value, a first decision is made to start with the first test 3 or the second test 4. The method 1 comprises a step of sorting eggs based on test sequence and performing different test sequences in parallel. The sorting after the egg assessment 11 of eggs based on test sequence facilitates to perform different test sequences in parallel. In turn, performing different test sequences in parallel, improves the throughput of the plurality of eggs 2 to be tested.

In case of performing different test sequences in parallel, it is useful for the method 1 to comprise synchronizing total lead times for test sequences, preferably all test sequences. This way it is possible to maintain batches of eggs before and after the method. These batches of eggs can then be incubated 12 as single stage process .This means that the eggs are maintained in a same or similar phase of development. The hatching of the eggs 5 will than still occur within an acceptable hatching window of normally about 48 hours.

When performing different test sequences in parallel, and synchronize these test sequence will result in incubating 12 and hatching of a batch that contains respective eggs that were subjected to different respective test sequences.

Fig. 2 shows a schematic overview of a second embodiment of the method according to the invention. in principle, only the most important differences with fig. 1 are clarified.

In this case, the test sequence starts with the first test 3. In other words, the step 6 of determining a test usability value for an individual egg does not precede the first test 3. Instead, the first test result provides a basis for the test usability value. Following the test usability value, a decision is made to perform the second test 4 or perform no further test at all. When the decision is made to perform no further test at all, and undetermined status or reject status will be assigned to the respective egg.

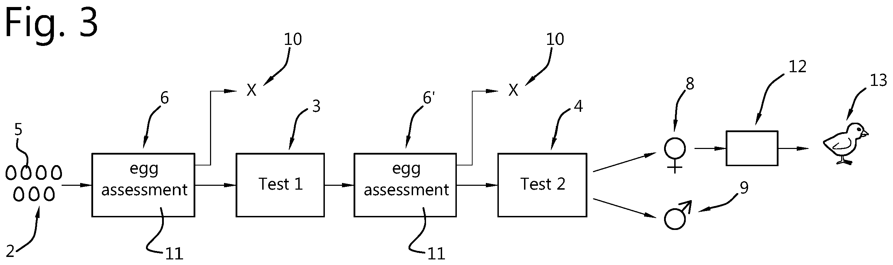

Fig. 3 shows a schematic overview of a third embodiment of the method according to the invention. In principle, only the most important differences with fig. 1 are clarified.

In this case, the test sequence starts with the egg assessment 11. The step 6 of determining a test usability value for an individual egg thus precedes the first test 3 as well as the second test 4 if required. Instead, the first test result provides a basis for the test usability value. Following the test usability value, a decision is made to perform the first test 3 or perform no further test at all. When the decision is made to perform no further test at all, and undetermined status or reject status will be assigned to the respective egg. When the first test 3 does not deliver a satisfactory result in terms of accuracy of the outcome, the first test result can provide a basis as well for the test usability value. In that case the test usability value is processed into an updated test usability value 6' for an individual egg 5 also based on the first result. Following the updated test usability value, a decision is made to perform the second test 4 or perform no further test at all. Thus, the step 6' of determining the updated test usability value for an individual egg is based on the first result as well. When the decision is made to perform no further test at all, and undetermined status or reject status will be assigned to the respective egg.

The present invention has been described above with reference to a number of exemplary embodiments as shown in the drawings. Modifications and alternative implementations of some parts or elements are possible, and are included in the scope of protection as defined in the appended claims.

## Claims

1. A method for determining the gender of a birds in ovo of a plurality of eggs, comprising a first test, and a second test separate and different from the first test, each of the first and second test comprising the steps of examining a bird egg and determining the gender of the bird within the egg, and wherein the method further comprises the step of determining a test usability value for each individual egg of the plurality of eggs and deciding a test sequence for each individual egg based on the test usability value, the test sequence comprising one or both of the first and second test, and separating said plurality of eggs into at least a first and second subset of eggs based on gender, wherein the determining the test usability value for each individual egg of the plurality of eggs comprises assessing an individual egg for providing an egg assessment and wherein the test usability value for the individual egg is at least based on the egg assessment, and wherein the step of assessing an individual egg comprises one or more of; Colori metering, weighing, temperature measuring, imaging, candling, egg shell thickness measurement, the method being **characterised in that** the first and second test at least differ in working principle.

2. Method according to claim 1, wherein the step of determining a test usability value for an individual egg precedes the first test and the second test, and a decision is made to perform the first test or the second test or reject the individual egg based on the usability value, or the step of determining a test usability value for an individual egg precedes the second test, and a decision is made to perform the second test or reject the individual egg based on the usability value.

3. Method according to a preceding claim, wherein the first test provides a first result and the second test provides a second result and wherein the determining the test usability value for an individual egg is at least based on the first result, and a decision is made to perform the second test or not is based on the test usability value.

4. Method according to a preceding claim, comprising updating the test usability value to an updated test usability value based on the first result and/or the second test result.

5. Method according to a preceding claim, comprising a step of sorting eggs based on test sequence and performing different test sequences in parallel.

6. Method according to a preceding claim, wherein the first and second test differ in one or more of; cost, consumables, cycle time, and lead time.

7. Method according to a preceding claim, wherein the first test and/or the second comprise one or more of spectroscopy, component analyses, and image analysis.

8. Method according to a preceding claim, wherein the first test comprises spectroscopy and the second test comprises PCR.

9. Method according to a preceding claim, wherein the first and/or second test comprises detecting the presence or absence of an elevated level of a sex-related component in the bird egg, in particular in the extra-embryonic fluid of the bird egg; and determining the gender of the bird within the egg from the presence of an elevated level of said sex-related hormone in the extra-embryonic fluid.

10. Method according to a preceding claim, wherein the first and/or second test comprises examining DNA for determining the gender of the bird within the egg.

11. Method according to a preceding claim, comprising the step of sampling an egg to provide an amount of extra-embryonic fluid, and using the amount for the first and second test.

12. Method according to a preceding claim, comprising synchronizing total lead times for test sequences for maintaining batches of eggs before and after the method.

13. Method according to claim 12, comprising hatching a batch of eggs, wherein the batch contains respective eggs that were subjected to different respective test sequences.

14. Method according to a preceding claim, comprising performing the method with embryonated eggs after day 4 in their development, in particular at about day 9 in their development.

15. Method according to a preceding claim, wherein the first test and the second test are executed on different moments in the development of a bird egg, in particular for a chicken egg the first test is executed before day 11 and the second test is executed after day 11 in the development of the chicken egg, more particular the second test is executed after day 16 in the development of the chicken egg, in particular after day 18 in the development of the chicken egg.

16. Method according to a preceding claim, wherein the first test and the second test are executed on different moments in the development of a bird egg, and in an embodiment, a time lapse between the first test and the second test exceeds 24 hours.

17. Method according to a preceding claim, comprising climatizing a test environment, comprising maintaining the test environment at a temperature of about 35°.

## Patentansprüche

1. Verfahren zur Bestimmung des Geschlechts eines Vogels im Ei aus einer Vielzahl von Eiern, das einen ersten Test und einen zweiten Test umfasst, der von dem ersten Test getrennt ist und sich von ihm unterscheidet, wobei sowohl der erste als auch der zweite Test die Schritte des Untersuchens eines Vogeleis und des Bestimmens des Geschlechts des Vogels in dem Ei umfasst, und wobei das Verfahren ferner den Schritt des Bestimmens eines Testnutzbarkeitswerts für jedes einzelne Ei der Vielzahl von Eiern und des Festlegens einer Testsequenz für jedes einzelne Ei basierend auf dem Testnutzbarkeitswert umfasst, wobei die Testsequenz den ersten und/oder den zweiten Test und das Aufteilen der besagten Vielzahl von Eiern in mindestens eine erste und zweite Untergruppe von Eiern basierend auf dem Geschlecht umfasst, wobei das Bestimmen des Testnutzbarkeitswerts für jedes einzelne Ei der Vielzahl von Eiern das Bewerten eines einzelnen Eis zum Bereitstellen einer Eierbewertung umfasst und wobei der Testnutzbarkeitswert für das einzelne Ei zumindest auf der Eierbewertung basiert, und wobei der Schritt des Bewertens eines einzelnen Eis eines oder mehreres von Folgendem umfasst: Kolonmessung, Wiegen, Temperaturmessung, Bildgebung, Durchleuchten und/oder Messung der Eierschalendicke, wobei das Verfahren **dadurch gekennzeichnet ist, dass** sich der erste und zweite Test zumindest im Funktionsprinzip unterscheiden.

2. Verfahren nach Anspruch 1, wobei der Schritt des Bestimmens eines Testnutzbarkeitswerts für ein einzelnes Ei dem ersten Test und dem zweiten Test vorausgeht und basierend auf dem Nutzbarkeitswert entschieden wird, den ersten Test oder den zweiten Test durchzuführen oder das einzelne Ei abzulehnen, oder wobei der Schritt des Bestimmens eines Testnutzbarkeitswerts für ein einzelnes Ei dem zweiten Test vorausgeht und basierend auf dem Nutzbarkeitswert entschieden wird, den zweiten Test durchzuführen oder das einzelne Ei abzulehnen.

3. Verfahren nach einem vorhergehenden Anspruch, wobei der erste Test ein erstes Ergebnis bereitstellt und der zweite Test ein zweites Ergebnis bereitstellt und wobei das Bestimmen des Testnutzbarkeitswerts für ein einzelnes Ei zumindest auf dem ersten Ergebnis basiert und die Entscheidung, den zweiten Test durchzuführen oder nicht, auf dem Testnutzbarkeitswert basiert.

4. Verfahren nach einem vorhergehenden Anspruch, das das Aktualisieren des Testnutzbarkeitswerts auf einen aktualisierten Testnutzbarkeitswert basierend auf dem ersten Ergebnis und/oder dem zweiten Testergebnis umfasst.

5. Verfahren nach einem vorhergehenden Anspruch, das einen Schritt des Sortierens der Eier basierend auf der Testsequenz und das parallele Durchführen verschiedener Testsequenzen umfasst.

6. Verfahren nach einem vorhergehenden Anspruch, wobei sich der erste und zweite Test in einem oder mehreren von Folgendem unterscheiden: den Kosten, den Verbrauchsmaterialien, der Zykluszeit und/oder der Vorlaufzeit.

7. Verfahren nach einem vorhergehenden Anspruch, wobei der erste und/oder der zweite Test eine Spektroskopie, eine Komponentenanalyse und/oder eine Bildanalyse umfassen.

8. Verfahren nach einem vorhergehenden Anspruch, wobei der erste Test eine Spektroskopie und der zweite Test eine PCR umfasst.

9. Verfahren nach einem vorhergehenden Anspruch, wobei der erste und/oder zweite Test das Erkennen des Vorhandenseins oder Fehlens eines erhöhten Gehalts einer geschlechtsbezogenen Komponente in dem Vogelei, insbesondere in der extraembryonalen Flüssigkeit des Vogeleis; und das Bestimmen des Geschlechts des Vogels in dem Ei anhand des Vorhandenseins eines erhöhten Gehalts des besagten geschlechtsbezogenen Hormons in der extraembryonalen Flüssigkeit umfassen.

10. Verfahren nach einem vorhergehenden Anspruch, wobei der erste und/oder zweite Test das Untersuchen der DNA zum Bestimmen des Geschlechts des Vogels in dem Ei umfassen.

11. Verfahren nach einem vorhergehenden Anspruch, das den Schritt des Entnehmens einer Probe aus einem Ei umfasst, um eine Menge an extraembryonaler Flüssigkeit zu gewinnen, und das Verwenden der Menge für den ersten und zweiten Test.

12. Verfahren nach einem vorhergehenden Anspruch, das das Synchronisieren der gesamten Vorlaufzeiten für Testsequenzen zum Aufrechterhalten von Eierchargen vor und nach dem Verfahren umfasst.

13. Verfahren nach Anspruch 12, das das Ausbrüten einer Charge von Eiern umfasst, wobei die Charge entsprechende Eier enthält, die unterschiedlichen entsprechenden Testsequenzen unterzogen wurden.

14. Verfahren nach einem vorhergehenden Anspruch, das das Durchführen des Verfahrens mit bebrüteten Eiern nach dem 4. Entwicklungstag umfasst, insbesondere um den 9. Entwicklungstag herum.

15. Verfahren nach einem vorhergehenden Anspruch, wobei der erste Test und der zweite Test zu unterschiedlichen Zeitpunkten in der Entwicklung eines Vogeleis durchgeführt werden, wobei insbesondere bei einem Hühnerei der erste Test vor dem 11. Tag und der zweite Test nach dem 11. Tag der Entwicklung des Hühnereis durchgeführt wird, wobei genauer gesagt der zweite Test nach dem 16. Entwicklungstag des Hühnereis durchgeführt wird, insbesondere nach dem 18. Entwicklungstag des Hühnereis.

16. Verfahren nach einem vorhergehenden Anspruch, wobei der erste Test und der zweite Test zu unterschiedlichen Zeitpunkten in der Entwicklung eines Vogeleis durchgeführt werden und in einer Ausführungsform eine Zeitspanne zwischen dem ersten Test und dem zweiten Test 24 Stunden überschreitet.

17. Verfahren nach einem vorhergehenden Anspruch, das das Klimatisieren einer Testumgebung umfasst, wobei die Testumgebung auf einer Temperatur von etwa 35° gehalten wird.

## Revendications

1. Procédé pour déterminer le sexe d'un oiseau dans l'oeuf pour une pluralité d'oeufs , comprenant un premier test et un deuxième test séparé et différent du premier test, chacun des premier et deuxième tests comprenant les étapes consistant à examiner un œuf d'oiseau et déterminer le sexe de l'oiseau contenu dans l'œuf, et où le procédé comprend en outre l'étape consistant à déterminer une valeur d'utilisabilité du test pour chaque œuf individuel de la pluralité d'œufs et décider une séquence de test pour chaque œuf individuel sur la base de la valeur d'utilisabilité du test, la séquence de test comprenant l'un ou les deux des premier et deuxième tests, et séparer ladite pluralité d'œufs en au moins un premier et un deuxième sous-ensemble d'œufs en fonction du sexe, où la détermination de la valeur d'utilisabilité du test pour chaque œuf individuel de la pluralité d'œufs comprend l'évaluation d'un œuf individuel afin de fournir une évaluation de l'œuf et où la valeur d'utilisabilité du test pour l'œuf individuel est au moins basée sur l'évaluation de l'œuf, et où l'étape d'évaluation d'un œuf individuel comprend une ou plusieurs des opérations suivantes : la mesure de la couleur, la pesée, la mesure de la température, l'imagerie, le mirage, la mesure de l'épaisseur de la coquille, le procédé étant **caractérisé en ce que** le premier et le deuxième test diffèrent au moins par leur principe de fonctionnement.

2. Procédé selon la revendication 1, où l'étape consistant à déterminer une valeur d'utilisabilité du test pour un œuf individuel précède le premier test et le deuxième test, et une décision est prise d'effectuer le premier test ou le deuxième test ou de rejeter l'œuf individuel sur la base de la valeur d'utilisabilité, ou bien l'étape consistant à déterminer une valeur d'utilisabilité du test pour un œuf individuel précède le deuxième test, et une décision est prise d'effectuer le deuxième test ou de rejeter l'œuf individuel sur la base de la valeur d'utilisabilité.

3. Procédé selon l'une des revendications précédentes, où le premier test fournit un premier résultat et le deuxième test fournit un deuxième résultat, et où la détermination de la valeur d'utilisabilité du test pour un œuf individuel est au moins basée sur le premier résultat, et une décision est prise d'effectuer ou non le deuxième test sur la base de la valeur d'utilisabilité du test.

4. Procédé selon l'une des revendications précédentes, comprenant la mise à jour de la valeur d'utilisabilité du test en une valeur d'utilisabilité du test mise à jour sur la base du premier résultat et/ou du résultat du deuxième test.

5. Procédé selon l'une des revendications précédentes, comprenant une étape consistant à trier des œufs en fonction de la séquence de test et à effectuer différentes séquences de test en parallèle.

6. Procédé selon l'une des revendications précédentes, où le premier et le deuxième test diffèrent par un ou plusieurs des éléments suivants : coût, consommables, durée du cycle et délai d'exécution.

7. Procédé selon l'une des revendications précédentes, où le premier test et/ou le deuxième test comprennent un ou plusieurs des éléments suivants : spectroscopie, analyses de composants et analyse d'images.

8. Procédé selon l'une des revendications précédentes, où le premier test comprend la spectroscopie et le deuxième test comprend la PCR.

9. Procédé selon l'une des revendications précédentes, où le premier et/ou le deuxième test comprend la détection de la présence ou de l'absence d'un taux élevé d'un composant lié au sexe dans l'œuf d'oiseau, en particulier dans le liquide extra-embryonnaire de l'œuf d'oiseau ; et la détermination du sexe de l'oiseau dans l'œuf à partir de la présence d'un taux élevé de ladite hormone liée au sexe dans le liquide extra-embryonnaire.

10. Procédé selon l'une des revendications précédentes, où le premier et/ou le deuxième test comprend l'examen de l'ADN pour déterminer le sexe de l'oiseau dans l'œuf.

11. Procédé selon l'une des revendications précédentes, comprenant l'étape consistant à prélever un échantillon d'un œuf pour obtenir une quantité de liquide extra-embryonnaire, et utiliser cette quantité pour le premier et le deuxième test.

12. Procédé selon l'une des revendications précédentes, comprenant la synchronisation des délais d'exécution totaux pour les séquences de test afin de maintenir les lots d'œufs avant et après le procédé.

13. Procédé selon la revendication 12, comprenant l'incubation d'un lot d'œufs, où le lot contient des œufs respectifs qui ont été soumis à des séquences de test respectives différentes.

14. Procédé selon l'une des revendications précédentes, comprenant la mise en œuvre du procédé avec des œufs embryonnés après le 4^{e} jour de leur développement, en particulier vers le 9^{e} jour de leur développement.

15. Procédé selon l'une des revendications précédentes, où le premier test et le deuxième test sont effectués à des moments différents du développement d'un œuf d'oiseau, en particulier pour un œuf de poule le premier test est effectué avant le 1^{er} jour et le deuxième test est effectué après le 1^{e} jour du développement de l'œuf de poule, plus particulièrement le deuxième test est effectué après le 16^{e} jour du développement de l'œuf de poule, en particulier après le 18^{e} jour du développement de l'œuf de poule.

16. Procédé selon l'une des revendications précédentes, où le premier test et le deuxième test sont effectués à des moments différents du développement d'un œuf d'oiseau, et dans un mode de réalisation, un intervalle de temps entre le premier test et le deuxième test dépasse 24 heures.

17. Procédé selon l'une des revendications précédentes, comprenant la climatisation d'un environnement de test, comprenant le maintien de l'environnement de test à une température d'environ 35°C.
